# EUROPEAN PATENT SPECIFICATION

(11) **EP 2 236 596 B1**
(45) Date of publication and mention of the grant of the patent: **25.07.2012**
(21) Application number: 09157334.5
(22) Date of filing: 03.04.2009
(51) Int. Cl.: C12M 1/34, G01N 15/14

(54) **High fidelity colour imaging of microbial colonies**
Farbbildgebung von Mikrobenkolonien mit hoher Wiedergabetreue
Imagerie couleur haute fidélité de colonies microbiennes

(43) Date of publication of application: 06.10.2010
(73) Proprietor: Synoptics Limited, Cambridge Cambridgeshire CB4 1TF (GB)
(72) Inventor: Atkin, Philip, Cambridge, Cambridgeshire CB23 1LP (GB); Hayden-Wright, Alasdair Graham, Cambridge, Cambridgeshire CB4 3PE (GB)
(74) Representative: Brunner, Michael John

(56) References cited:
- EP-A- 1 061 127
- DE-A1- 2 455 870
- US-A- 4 456 380
- US-A1- 2004 102 903

## Description

### Background to the Invention

The invention relates to methods and systems for imaging microbial colonies. In particular, the invention concerns a method and an associated system for imaging such colonies in high fidelity and in colour.

The identification and enumeration of microbial colonies is important in many fields such as the food and water industries, pharmaceutical production and medical diagnosis. In the most common technique, a sample is plated onto the surface of an agar medium in a sample plate, such as a Petri dish. The dish is incubated and the bacteria encouraged to grow whilst consuming the agar. Each bacterium in the original sample multiplies and gives rise to a visible 'colony' on the plate; counting these colonies thereby provides an indication of the level of microbial contamination of the original sample. Automatic, computer-based instruments ("automatic colony counters", such as the ProtoCOL and aCOLyte instruments from Synbiosis) are increasingly used to replace human visual interpretation of such plates. A parallel innovation is the introduction of chromogenic media (such as those produced by BD Diagnostics and bioMérieux), in which different varieties of microbes turn different colours, facilitating identification and allowing more than one population of microbes to be enumerated on a single plate.

In order to produce an automatic instrument to record, document and analyse pictures of such Petri dishes, it is necessary to provide a camera and an illumination system that can accurately and faithfully record the features of the dish and its contents. This involves solving a number of technical challenges.

In order to achieve accurate colour capture, each colour pixel in a captured image of the dish and its contents (i.e. the microbial colonies) is 'classified' in order to decide whether it belongs to a particular colour class (range of colours) that correspond to a particular type of organism or other type of feature in the sample. For example, "lurid green pixels indicate cryptosporidium".

Once the colour of the sample at every point has been accurately determined by means of an intensity value of, for example, between 0 and 255 for each of two or more spectral ranges ("colours"), automatic classification of each pixel may be computed by means of a conventional algorithm applied to the digital intensity values measured for each illumination colour at each pixel. For example, a simple system may be "trained" to classify pixels where the red and blue values are below 10 and the green values are between 150 and 200 as being indicative of the presence of a particular bacterium. Subsequently, a further algorithm may detect adjacent pixels having an identical class as belonging to the same 'colony' of bacteria, and is therefore counted as a single entity.

Currently, colour images of such colonies are captured using conventional colour cameras. Conventional colour cameras use a monochrome sensor (that is, one responsive to all frequencies of light) covered by a filter mosaic called a Bayer filter. From each group of 4 pixels (generally two green, one red, one blue), software is used to interpolate 12 pixel values (R, G and B for each of the 4 pixel locations). Due to the lack of full information, this interpolation process is imperfect. In this application, these imperfections can lead to significant errors in colour information around sharp boundaries in intensity. Put simply, a sharp edge may 'sparkle' with incorrect colours, which in turn leads to incorrect classification.

Alternative colour image capture devices, such as the Foveon X3 (TM) sensor from Foveon, Inc. or 3-chip cameras, are very much more expensive than a conventional colour camera and are accordingly not practical for this application.

Another challenge is that inexpensive lenses tend to suffer from chromatic aberration; in particular lateral or transverse chromatic aberration, which leads to images formed by light of different frequencies having a different magnification. This effect can also lead to incorrect colours ("fringes") around the sharp boundaries of objects, and in turn to incorrect classification.

For efficient workflow, easy access by the operator to the sample area is important. However, open access can lead to ambient light having a strong effect on the captured image; this can also lead to failure to detect features in the image or to incorrect colour classification.

It is an object of the invention to provide a method and associated system that addresses these challenges and shortcomings of existing techniques. In particular, it is desired to provide accurate, high fidelity colour capture in a cost-effective manner.

In US 2004/0102903, there is disclosed a method as defined by the preamble of the accompanying claim 1 and a system as defined by the preamble of the accompanying claim 11.

### Summary of the Invention

According to a first aspect of the invention, there is provided a method of imaging microbial colonies in a sample, comprising the steps of:
illuminating the colonies with light of different colours in turn;
for each illumination colour, capturing a monochrome image of the colonies;
combining the respective monochrome images into a composite colour image of the microbial colonies. and calibrating for each illumination colour, the effect of chromatic aberration of the lens and correction for that effect.

For each colour, a light source of that colour is turned on and a monochrome image is immediately captured. By capturing a series of monochrome images with the sample under differently coloured illumination, each such image can form one 'channel' of a composite colour image. This avoids the need for software interpolation of the pixel information and produces highly accurate colour information in each pixel at the full spatial resolution and full sensitivity of the (unfiltered) sensor which in turn leads to the elimination of any colour 'sparkle' due to imperfections in such interpolation. A monochrome camera (i.e. one without a filter mosaic) including a lens is used. The method further comprises the steps of calibrating, for each illumination colour, the effect of chromatic aberration of the lens, and correcting for that effect.

By calibrating the effect of chromatic aberration of the lens (and potentially other optical elements) for each illumination colour, and correcting for it, the accuracy of the colour information may be further enhanced through the elimination of any difference in magnification of the features of the sample when sensed by each colour, thereby eliminating the colour fringes. This means that less expensive lenses can be used, even when working at high camera resolutions.

The step of illuminating may include illumination with red, green and blue light sources and/or illumination with light sources outside the spectrum visible to humans, i.e. infra-red or ultra-violet.

Where the light sources comprise red, green and blue lights, the individual red, green and blue pixels at each location may be combined to form each RGB colour 'pixel' of a 'true colour' image. Conversely, there is no need to limit the illumination to only three colours; more visible wavelengths and indeed those beyond the visible (infra-red and ultra-violet) are also possible with the same scheme. Colour classification and chromatic aberration correction techniques can easily be extended to combine all the resulting information (chromatic aberration in a typical lens is particularly severe at extreme wavelengths).

The illumination may be provided by LEDs (Light-Emitting Diodes).

Since LEDs can be turned on or off effectively instantaneously, it is possible to perform a complete acquisition sequence very rapidly, without waiting for tubes to stop or start glowing, for example. They also emit a narrow band of frequencies (colours) and therefore the illumination is tightly controlled.

According to one aspect of the invention, the different illumination colours are selected such that the resultant composite image is a true colour image.

As discussed above, it is not only a combination of RGB light sources that can be used to provide a true colour composite image; alternative illumination schemes are possible.

The step of capturing a monochrome image for each illumination colour may include capturing multiple individual images for each colour and averaging or summing the individual images.

By averaging or summing the individual images, the effects of noise can be reduced.

Optionally, the method may further comprise the step of capturing an additional image of the microbial colonies with no illumination to determine the amount of ambient light on the sample. If the amount of ambient light is above a threshold level, the method may further comprise the step of warning the user. If the amount of ambient light is below a threshold level, the method may further comprise the step of correcting each captured image by subtracting the ambient light image from the respective captured image.

By capturing an additional image with the illumination switched off the level of ambient light on the sample can be determined. From the level of light in this image (such as the peak or average) it can be decided whether to warn the user that the ambient light level is too high (i.e. above a threshold level) for accurate imaging. If so, the user may then choose to shield the system or to close an orifice to block out the ambient light. If the level of ambient light falls below this threshold, the 'ambient' image can instead be used to correct each captured image by subtracting the ambient light image from that captured image.

Optionally, the method may further comprise the step of calibrating for varying intensities of illumination provided by each colour, said calibration comprising:
providing a fixed calibration target in the vicinity of the sample;
establishing a reference intensity for the target for each illumination colour;
capturing with each monochrome image an image of the target;
determining a ratio and/or offset of the intensity of the captured image of the target from the reference intensity; and
adjusting the captured image on the basis of the ratio and/or offset.

Variations in the intensity of the illuminations provided by each colour (e.g. fluctuations in the junction temperature of the LEDs where these comprise the light sources) lead to instability in the values in the digital images. It is important that the overall system continue to deliver the same values in successive digital images of an unchanging sample. This is most conveniently achieved by placing a fixed target or targets in the field of view so that they may be captured alongside the sample plate each time. A calibration establishes a reference intensity for each target, for each colour channel. Since the effect of a variation in illumination intensity for each colour channel is to vary the overall 'gain' for that colour, to correct this variation it is only necessary to multiply each colour channel by a constant so as to restore the measured intensity of the target to the reference intensity; all other pixels will then have the correct intensity. With two or more targets it is also possible to correct for an offset in the camera's response to light (such that the digital output in the absence of light is non-zero).

According to another aspect of the invention, there is provided a microbial colony imaging system comprising:
an illumination system adapted to illuminate microbial colonies in a sample with light of different colours in turn;
an image capture device adapted to capture a monochrome image of the colonies for each illumination colour; and
a processor to combine the respective monochrome images into a composite colour image of the microbial colonies, configured to calibrate, for each illumination colour, the effect of chromatic aberration of the lens, and to correct for that effect.

The image capture system comprises a monochrome camera (that is, one sensitive to all light frequencies over the required spectrum) having a lens. The processor is configured to calibrate, for each illumination colour, the effect of chromatic aberration of the lens, and to correct for that effect.

Typically, the illumination system comprises red, green and blue light sources, which are preferably LEDs.

The system may be adapted to carry out any of the methods defined above.

The system may further include one of an opaque, diffusing or transparent disc located in a light path between the illumination system and the sample, each disc having a unique pattern of small holes that is detectable by the image capture device.

Each unique pattern of small holes shows up in the captured images so that software associated with the processor can thus verify that the correct disc for the type of sample has been installed.

### Brief Description of the Drawings

The invention will now be described, by way of example, with reference to the accompanying drawings, in which:
Figure 1 illustrates schematically a cross-sectional side elevation of microbial colony imaging system embodying the invention; and
Figure 2 illustrates schematically the field of view of a camera of the system of the invention (i.e. a plan view of a microbial colony sample placed on a blanking disc).

### Detailed Description

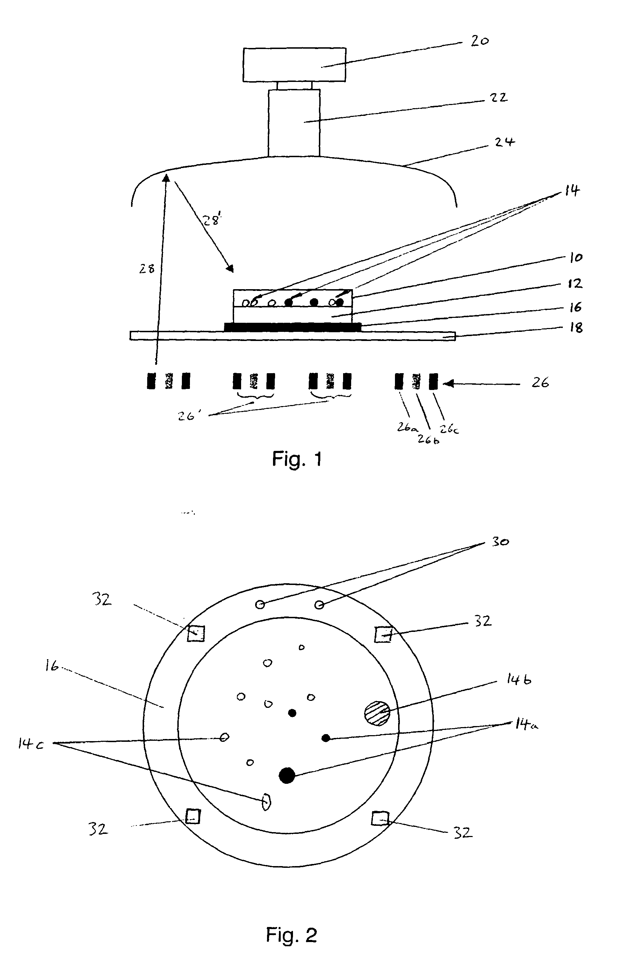

Figure 1 illustrates, schematically, a system for imaging microbial colonies. A conventional sample plate 10, such as a Petri dish, having a base layer of agar medium 12 has previously had a sample plated onto the surface of that agar medium and been incubated so that each bacterium in the original sample has multiplied and given rise to a visible colony 14. The sample plate 10 is supported by an opaque blanking disc 16. A diffuser 18 underlies the blanking disc 16.

A monochrome camera 20 (i.e. one without a filter mosaic) having a lens 22 is disposed centrally above the sample plate 10, such that the field of view of the camera encompasses the sample plate 10 and the blanking disc 16. Attached to the camera and lens combination is a curved reflector 24.

Light sources 26 of different colours are disposed below the diffuser 18. Light rays 28 from the light sources 26 pass up through the diffuser 18 and are reflected by the reflector 24 to illuminate the sample plate 10 and the microbial colonies 14 contained therein. Microbial colonies often grow noticeably out of the planar surface of the plate; they are three-dimensional. This can lead to highlights on the colonies themselves unless the illumination is very diffuse. The described arrangement ensures that the sample plate 10 is dififusely illuminated from a wide range of directions, thereby avoiding point sources where possible to prevent such specular highlights. The range of directions is constrained, however, to avoid direct reflection of the incident light 28' into the camera 20 by the surface of the sample.

Alternatively, rather than blocking direct illumination of the sample plate 10 from below with the blanking disc 16 and reflecting the light from above using the curved reflector 24, the blanking disc 16 could be replaced by a different disc that allows light to pass through, thereby illuminating the sample plate 10 directly from below. The diffuser 18 is optional. Hence, for different types of sample, different discs may be inserted into the light path to pass, block or diffuse the light. Although described as discs, it will be understood that the shape of these light path affecting features need not be circular.

Thus, according to the type of sample being imaged, it may be necessary to illuminate it in various ways by choosing an appropriate blanking disc, transparent disc or diffusing disc 16. In regulated environments (such as pharmaceutical manufacturing) it is important to verify that the correct imaging configuration has been selected.

Initially, the system is not illuminated. A first light source 26a of a first colour is then turned on and a monochrome image is immediately captured by the camera 20. That first light source 26a is subsequently turned off and a second light source 26b of a second colour is turned on, a second monochrome image being captured by the camera 20. The process is repeated with a third light source 26c. Hence, for each colour, the light source 26a; 26b; 26c of that colour is turned on and a monochrome image is immediately captured by the camera 20.

The respective monochrome images are input to a processor (not shown), where the inputs are combined to form a composite colour image. Hence, each such monochrome image can be considered as forming one 'channel' of the composite colour image.

There may be red, green and blue light sources 26a, 26b and 26c; whereby the individual red, green and blue pixels at each location from the respective captured monochrome images are combined to form each RGB colour 'pixel' of a 'true colour' image. This avoids the need for software interpolation of the pixel information and produces highly accurate colour information in each pixel at the full spatial resolution and full sensitivity of the (unfiltered) sensor.

There is no need to limit the illumination to only three colours; more visible wavelengths and indeed those beyond the visible (IR and UV) are also possible. Hence, it is not only a combination of RGB light sources that can be used to provide a true colour composite image.

The light sources preferably comprise sets of differently coloured individual light sources 26a, 26b, 26c, which may be arranged in groups 26'. Since light sources of a particular colour (e.g. blue) may not be as intense as those of other colours, more light sources of that less intense colour may be provided. Preferably, the light sources comprise LEDs (Light-Emitting Diodes). Since LEDs can be turned on or off effectively instantaneously, it i s possible to perform a complete acquisition sequence very rapidly, without waiting for tubes to stop or start glowing, for example.

The effects of noise can be reduced by taking multiple individual monochrome images for each colour of illumination and averaging or summing.

The resultant composite image may then be analysed using conventional software to 'classify' each colour pixel in the image in order to decide whether it belongs to a particular colour class (range of colours) that correspond to a particular type of organism or other type of feature in the sample, as described above. Once the colour of the sample at every point has been accurately determined by means of an intensity value of, for example, between 0 and 255 for each of two or more spectral ranges ("colours"), automatic classification of each pixel may be computed by means of a conventional algorithm applied to the digital intensity values measured for each illumination colour at each pixel. For example, a simple system may be "trained" to classify pixels where the red and blue values are below 10 and the green values are between 150 and 200 as being indicative of the presence of a particular bacterium. Subsequently, a further algorithm may detect adjacent pixels having an identical class as belonging to the same 'colony' of bacteria, and is therefore counted as a single entity. In this manner, the software is able to detect and distinguish between colonies 14a of one bacterium from colonies 14b of another bacterium.

The software may further be able to distinguish other features present in the image, such as to identify the type of disc 16 (opaque, diffusing or transparent) that is present between the light sources 26 and the sample plate 10. In particular, each different type of disc may contain a unique pattern of small holes 30 which show up in the image. The software can thus verify that the correct disc for the type of sample has been installed.

An additional image may be captured with the light sources 26 switched off to determine the level of ambient light on the sample. From the level of light in this 'ambient' image (such as the peak or average) it can be decided whether to warn the user that the ambient light level is too high (i.e. above a threshold level) for accurate imaging. If the level of ambient light is beyond the predefined threshold, the user may then choose to shield the system or to close an orifice to block out the ambient light.

Typically, the sample-containing sample plate 10 may be contained within a chamber (not shown) having access doors that can be closed to block out ambient light. Hence, one response to the ambient light level being above the threshold could be to close such chamber doors.

If, conversely, the level of ambient light is below this threshold, the 'ambient' image can instead be used to correct each captured image by subtracting the ambient light image from that captured image.

For efficient workflow, easy access by the operator to the sample area is important. However, open access can lead to ambient light having a strong effect on the captured image; this can also lead to failure to detect features in the image or to incorrect colour classification. The possibility of detecting high levels of ambient light and correcting the effects of moderate levels means there is less need for a closeable door on the sample chamber; this in turn leads to greater convenience for the user. Similarly, there is less need for high levels of instrument illumination in order to 'drown out' the effects of ambient light.

The ambient light level can be assessed from a single monochrome image captured when all illumination is off. This single image can be used to correct each image captured with a single colour switched on, since it affects each such frame equally. To do the same with a conventional camera would require a full-colour image to be captured with the illumination off.

Similar schemes to those discussed above in connection with identifying the type of disc 16 present between the light sources 26 and the sample plate 10 would be possible to verify, for example, that the chamber doors (where present) are closed. The brightness of some light sources 26 varies significantly according to their temperatures. Where more than one light source 26 is used, as in the present invention, this can lead to variation in the relative intensity of the sources.

Variations in the intensity of the illuminations provided by each colour (e.g. fluctuations in the junction temperature of the LEDs where these comprise the light sources 26) lead to instability in the values in the digital images. It is important that the overall system continue to deliver the same values in successive digital images of an unchanging sample. This is most conveniently achieved by placing a fixed target or targets 32 in the field of view so that they may be captured alongside the sample plate 10 each time.

A calibration establishes a reference intensity for each target 32, for each colour channel corresponding to each colour of illumination. Since the effect of a variation in illumination intensity for each colour channel is to vary the overall 'gain' for that colour, to correct this variation it is only necessary to multiply each colour channel by a constant so as to restore the measured intensity of the target to the reference intensity; all other pixels will then have the correct intensity. With two or more targets 32 it is also possible to correct for an offset in the camera's response to light (such that the digital output in the absence of light is non-zero).

As discussed above, a disadvantage of relatively inexpensive lenses is that they are susceptible to chromatic aberration. The invention enables the calibration and correction of the effects of chromatic aberration of the lens individually for each colour of illumination. By calibrating the effect of chromatic aberration of the lens (and potentially other optical elements) for each illumination colour, and correcting for it, the accuracy of the colour information may be further enhanced, eliminating the colour fringes. This means that less expensive lenses can be used, even when working at high camera resolutions.

Accurate correction of chromatic aberration is easier than with a conventional colour camera, because the correction should preferably be applied before the interpolation/filtering process required by the Bayer filter is applied. This would be particularly complex because there is information about the (e.g. red) channel only at one in four pixel locations yet chromatic aberration shifts smaller than a single pixel need to be corrected.

The above-described colour classification and chromatic aberration correction techniques can easily be extended to illumination from more than three colours and indeed those beyond the visible spectrum, noting that chromatic aberration is particularly severe at extreme wavelengths.

Low-intensity light of all colours may be used to allow the sample to be imaged in monochrome to provide feedback to the operator as the sample plate 10 is positioned under the camera 20, and in order for the system to detect automatically when the user is changing or adjusting the disc 16.

For simpler sample types where only presence/absence detection is required (i.e. no colour classification), illumination could be chosen to be a single colour only or a fixed combination of illumination (white, for example).

## Claims

1. A method of imaging microbial colonies (14) in a sample, comprising the steps of:
illuminating the colonies (14) with light of different colours in turn;
for each illumination colour, capturing a monochrome image of the colonies by use of a monochrome camera (20) including a lens (22); and
combining the respective monochrome images into a composite colour image of the microbial colonies,
**characterised in that** the method further comprises the steps of calibrating, for each illumination colour, the effect of chromatic aberration of the lens (22), and correcting for that effect.

2. The method of claim 1, wherein the step of illuminating includes illumination with red, green and blue light sources.

3. The method of claim 1 or claim 2, wherein the step of illuminating includes illumination with light sources (26) outside the spectrum visible to humans.

4. The method of claim 1, 2 or 3, wherein the illumination is provided by LEDs (26a-c).

5. The method of any preceding claim, wherein the different illumination colours are selected such that the resultant composite image is a true colour image.

6. The method of any preceding claim, wherein the step of capturing a monochrome image for each illumination colour includes capturing multiple individual images for each colour and averaging or summing the individual images.

7. The method of any preceding claim, further comprising the step of capturing an additional image of the microbial colonies (14) with no illumination to determine the amount of ambient light on the sample.

8. The method of claim 7, further comprising the step of warning the user if the amount of ambient light is above a threshold level.

9. The method of claim 7, wherein, if the amount of ambient light is below a threshold level, the method further comprises the step of correcting each captured image by subtracting the ambient light image from the respective captured image.

10. The method of any preceding claim, further comprising the step of calibrating for varying intensities of illumination provided by each colour, said calibration comprising:
providing a fixed calibration target (32) in the vicinity of the sample;
establishing a reference intensity for the target (32) for each illumination colour;
capturing with each monochrome image an image of the target (32);
determining a ratio and/or offset of the intensity of the captured image of the target (32) from the reference intensity; and
adjusting the captured image on the basis of the ratio and/or offset.

11. A microbial colony imaging system comprising:
an illumination system (26) adapted to illuminate microbial colonies (14) in a sample with light of different colours in turn;
a monochrome camera (20) including a lens (22) adapted to capture a monochrome image of the colonies (14) for each illumination colour; and
a processor to combine the respective monochrome images into a composite colour image of the microbial colonies (14);
**characterised in that** the processor is configured to calibrate, for each illumination colour, the effect of chromatic aberration of the lens (22), and to correct for that effect.

12. The system of claim 11, wherein the illumination system comprises red, green and blue light sources (26a-c), which are preferably LEDs.

13. The system of claim 11, adapted to carry out the method of any of claims 1 to 10.

14. The system of claim 11 or 12, wherein the system further includes one of an opaque, diffusing or transparent disc (16) located in a light path (28) between the illumination system (26) and the sample, each disc having a unique pattern of small holes (30) that is detectable by the camera (20).

## Patentansprüche

1. Verfahren zur Bildgebung mikrobieller Kolonien (14) in einer Probe, folgende Schritte umfassend:
Beleuchten der Kolonien (14) mit Licht verschiedener Farben der Reihe nach;
für jede Beleuchtungsfarbe, Erfassen eines monochromen Bilds der Kolonien durch Verwendung einer monochromen Kamera (20) einschließlich einer Linse (22); und
Kombinieren der jeweiligen monochrome Bilder zu einem zusammengesetzten Farbbild der mikrobiellen Kolonien,
**dadurch gekennzeichnet, dass** das Verfahren weiter die Schritte Kalibrieren, für jede Beleuchtungsfarbe, des Effekts der chromatischen Aberration der Linse (22), und Korrektur für jenen Effekt umfasst.

2. Verfahren nach Anspruch 1, wobei der Beleuchtungsschritt die Beleuchtung mit roten, grünen und blauen Lichtquellen einschließt.

3. Verfahren nach Anspruch 1 oder Anspruch 2, wobei der Beleuchtungsschritt die Beleuchtung mit Lichtquellen (26) einschließt, die außerhalb des von Menschen sichtbaren Spektrums liegen.

4. Verfahren nach Anspruch 1, 2 oder 3, wobei die Beleuchtung durch LEDs (26a-c) bereitgestellt wird.

5. Verfahren nach einem beliebigen vorhergehenden Anspruch, wobei die verschiedenen Beleuchtungsfarben derart selektiert werden, dass das resultierende zusammengesetzte Bild ein wahres Farbbild ist.

6. Verfahren nach einem beliebigen vorhergehenden Anspruch, wobei der Schritt zum Erfassen eines monochromen Bilds für jede Beleuchtungsfarbe die Erfassung mehrfacher individueller Bilder für jede Farbe und Mittelung oder Summierung der individuellen Bilder einschließt.

7. Verfahren nach einem beliebigen vorhergehenden Anspruch, das weiter den Schritt der Erfassung eines zusätzlichen Bilds der mikrobiellen Kolonien (14) ohne Beleuchtung umfasst, um den die des Umgebungslichts auf der Probe zu ermitteln.

8. Verfahren nach Anspruch 7, das weiter den Schritt der Warnung des Anwenders umfasst, falls die Menge des Umgebungslichts über einem Schwellenwert liegt.

9. Verfahren nach Anspruch 7, wobei, falls die Menge von Umgebungslicht unter einem Schwellenwert liegt, das Verfahren weiter den Schritt der Korrektur jedes erfassten Bilds durch Subtrahieren des Umgebungslichts vom jeweiligen erfassten Bilds umfasst.

10. Verfahren nach einem beliebigen vorhergehenden Anspruch, das weiter den Schritt der Kalibrierung für variierende Beleuchtungsintensitäten umfasst, die von jeder Farbe bereitgestellt werden, wobei die besagte Kalibrierung umfasst:
Bereitstellen eines festen Kalibrierungsziels (32) in der Nähe der Probe;
Erstellen einer Referenzintensität für das Ziel (32) für jede Beleuchtungsfarbe;
Erfassen mit jedem monochromen Bild eines Bilds des Ziels (32);
Ermitteln eines Verhältnisses und/oder der Verschiebung (Offset) der Intensität des erfassten Bilds vom Ziel (32) von der Referenzintensität; und
Einstellen des erfassten Bilds auf der Basis des Verhältnisses und/oder der Verschiebung (Offset).

11. Bildgebungssystem für mikrobielle Kolonien, umfassend:
ein Beleuchtungssystem (26), das angepasst ist, die mikrobiellen Kolonien (14) in einer Probe der Reihe nach mit Licht verschiedener Farben zu beleuchten;
eine Monochrom-Kamera (20) einschließlich einer Linse (22), die angepasst ist, ein monochromes Bild der Kolonien (14) für jede Beleuchtungsfarbe zu erfassen; und
einen Prozessor zum Kombinieren der jeweiligen monochromen Bilder zu einem zusammengesetzten Farbbild der mikrobiellen Kolonien (14);
**dadurch gekennzeichnet, dass** der Prozessor konfiguriert ist, für jede Beleuchtungsfarbe, den Effekt chromatischer Aberration der Linse (22) zu kalibrieren und für jenen Effekt eine Korrektur vorzunehmen.

12. System nach Anspruch 11, wobei das Beleuchtungssystem rote, grüne und blaue Lichtquellen (26a-c) umfasst, die vorzugsweise LEDs sind.

13. System nach Anspruch 11, das angepasst ist, das Verfahren nach einem beliebigen der Ansprüche 1 bis 10 auszuführen.

14. System nach Anspruch 11 oder 12, wobei das eins von einer opaken, diffusen oder transparenten Scheibe (16) einschließt, die sich in einem Lichtweg (28) zwischen dem Beleuchtungssystem (26) und der Probe befindet, wobei jede Scheibe ein einzigartiges Muster von kleinen Löchern (30) aufweist, die von der Kamera (20) detektiert werden können.

## Revendications

1. Procédé d'imagerie de colonies microbiennes (14) dans un échantillon, comportant les étapes consistant à :
éclairer les colonies (14) à l'aide d'une lumière de différentes couleurs tour à tour ;
pour chaque couleur d'éclairage, capturer une image monochrome des colonies par l'utilisation d'une caméra monochrome (20) comprenant un objectif (22) ; et
combiner les images monochromes respectives en une image en couleur composée des colonies microbiennes,
**caractérisé en ce que** le procédé comprend par ailleurs les étapes consistant à calibrer, pour chaque couleur d'éclairage, l'effet d'aberration chromatique de l'objectif (22), et à corriger cet effet.

2. Procédé selon la revendication 1, dans lequel l'étape consistant à éclairer comprend l'éclairage à l'aide de sources lumineuses de couleur rouge, verte et bleue.

3. Procédé selon la revendication 1 ou la revendication 2, dans lequel l'étape consistant à éclairer comprend l'éclairage à l'aide de sources lumineuses (26) situées en dehors du spectre visible par l'oeil humain.

4. Procédé selon l'une quelconque des revendications 1, 2 ou 3, dans lequel l'éclairage est mis en oeuvre par des DEL (26a-c).

5. Procédé selon l'une quelconque des revendications précédentes, dans lequel les différentes couleurs d'éclairage sont sélectionnées de sorte que l'image composée résultante est une image en couleur naturelle.

6. Procédé selon l'une quelconque des revendications précédentes, dans lequel l'étape consistant à capturer une image monochrome pour chaque couleur d'éclairage comprend l'étape consistant à capturer de multiples images individuelles pour chaque couleur et l'étape consistant à faire la moyenne ou la somme des images individuelles.

7. Procédé selon l'une quelconque des revendications précédentes, comportant par ailleurs l'étape consistant à capturer une image supplémentaire des colonies microbiennes (14) sans éclairage pour déterminer la quantité de lumière ambiante sur l'échantillon.

8. Procédé selon la revendication 7, comportant par ailleurs l'étape consistant à signaler à l'utilisateur si la quantité de lumière ambiante est supérieure à un niveau seuil.

9. Procédé selon la revendication 7, dans lequel, si la quantité de lumière ambiante est inférieure à un niveau seuil, le procédé comporte par ailleurs l'étape consistant à corriger chaque image capturée en soustrayant l'image à lumière ambiante de l'image capturée respective.

10. Procédé selon l'une quelconque des revendications précédentes, comportant par ailleurs l'étape consistant à calibrer pour des intensités variables d'éclairage que procure chaque couleur, ledit calibrage comportant :
l'étape consistant à mettre en oeuvre une cible de calibrage fixe (32) à proximité de l' échantillon ;
l'étape consistant à établir une intensité de référence pour la cible (32) pour chaque couleur d'éclairage ;
l'étape consistant à capturer par chaque image monochrome une image de la cible (32) ;
l'étape consistant à déterminer un rapport et/ou un décalage de l'intensité de l'image capturée de la cible (32) à partir de l'intensité de référence ; et
l'étape consistant à ajuster l'image capturée en fonction du rapport et/ou du décalage.

11. Système d'imagerie de colonies microbiennes comportant :
un système d'éclairage (26) adapté pour éclairer des colonies microbiennes (14) dans un échantillon à l'aide d'une lumière de différentes couleurs tour à tour ;
une caméra monochrome (20) comprenant un objectif (22) adaptée pour capturer une image monochrome des colonies (14) pour chaque couleur d'éclairage ; et
un processeur pour combiner les images monochromes respectives en une image en couleur composée des colonies microbiennes (14) ;
**caractérisé en ce que** le processeur est configuré pour calibrer, pour chaque couleur d'éclairage, l'effet d'aberration chromatique de l'objectif (22), et pour corriger cet effet.

12. Système selon la revendication 11, dans lequel le système d'éclairage comporte des sources lumineuses de couleur rouge, verte et bleue (26a-c), qui sont de préférence des DEL.

13. Système selon la revendication 11, adapté pour réaliser le procédé selon l'une quelconque des revendications 1 à 10.

14. Système selon la revendication 11 ou la revendication 12, dans lequel le système comprend par ailleurs l'un d'un disque opaque, diffusant ou transparent (16) se trouvant dans un trajet de lumière (28) entre le système d'éclairage (26) et l'échantillon, chaque disque ayant une configuration unique de petits trous (30) que peut détecter la caméra (20).
